# EUROPEAN PATENT SPECIFICATION

(11) **EP 4 460 174 B1**
(45) Date of publication and mention of the grant of the patent: **25.02.2026**
(21) Application number: 23704053.0
(22) Date of filing: 05.01.2023
(51) Int. Cl.: A01G 33/00, C12N 1/12, C12R 1/89, A23L 29/00, A23L 17/60, A23L 33/105

(54) **CHLORELLA VULGARIS STRAIN WITH REDUCED CHLOROPHYLL CONTENT**
CHLORELLA-VULGARIS-STAMM MIT REDUZIERTEM CHLOROPHYLLGEHALT
SOUCHE DE CHLORELLA VULGARIS À TENEUR RÉDUITE EN CHLOROPHYLLE

(30) Priority: 05.01.2022 EP 22150374
(43) Date of publication of application: 13.11.2024
(73) Proprietor: Biotrino ApS, 2100 København Ø (DK)
(72) Inventor: MONTERO, Pablo Torres, 2800 Kongens Lyngby (DK); LU, Yi, 2800 Kongens Lyngby (DK); SIMONSEN, Henrik Toft, 2800 Kongens Lyngby (DK); HEDEGAARD, Søren, 2100 København Ø (DK)
(74) Representative: Inspicos P/S
(86) International application number: PCT/EP2023/050193
(87) International publication number: WO 2023/131656

(56) References cited:
- EP-A1- 3 622 828
- WO-A1-2020/105001
- WO-A1-2021/240232
- KR-A- 20140 114 274
- US-A1- 2011 256 282
- SHIN WON-SUB ET AL: "Truncated light-harvesting chlorophyll antenna size inChlorella vulgarisimproves biomass productivity", JOURNAL OF APPLIED PHYCOLOGY, KLUWER, DORDRECHT, NL, vol. 28, no. 6, 28 May 2016 (2016-05-28), pages 3193 - 3202, XP036116965, ISSN: 0921-8971, [retrieved on 20160528], DOI: 10.1007/S10811-016-0874-8

## Description

### FIELD OF THE INVENTION

The present invention relates to the fields of microbiology and human nutrition. In particular, the present invention relates to novel mutant *Chlorella vulgaris* strains having a unprecedented low chlorophyll content.

### BACKGROUND OF THE INVENTION

As the world human population continues to increase towards 10 billion people - who all need a daily intake of food (i.e. protein, carbohydrates, healthy fatty acids, vitamins, minerals, fibres) - and as CO₂ causes climate change, and a lack of water and diminished biodiversity puts pressure on food production, there is an ever-growing need for new sources of nutritious and tasty food, with a much lower total environmental impact/"footprint".

Considering that a major part of the global population is far from affluent, particularly food sources that are inexpensive to produce are, and will continue to be, in high demand.

Being by far the oldest foodstuff on planet earth, some 2 billion years older than the first plant, algae have for hundreds of years been known to man as a source of nutrition. Requiring only "sunlight and water" to grow, they are omnipresent on land and at sea and while certain types of algae, primarily macroalgae in the form of dried or fresh seaweed, are getting somewhat common for human consumption, the promise and potential of algae as a serious contender to address the global need for nutrition has yet in no way been realized. The reason for this is primarily that algae grown photosynthetically, e.g., in photo bioreactors or outdoor ponds, have a deep green colour (from chlorophyll) and a strong, unpleasant taste and smell, of rotten fish. This means that the commercially available food ingredients based on algae today are mostly relevant in small doses as a health supplement either in the form of pills or as "a teaspoon" mixed in a green "healthy smoothie" - and not as a main source of the daily nutrition needed by all humans. If these green algae are used in substantial quantities in recipes, e.g., in bread, pasta, pastry, dairy products, snacks, ice cream and the many other food products where they potentially could play a vital nutritious role, the end product will have a fishy taste and a dark green colour, very few, if any, consumers would like to buy - no matter what the price or "low footprint" marketing story told.

Out of the approx. over 20,000 species of algae known today several are used in foodstuff, most being macroalgae such as kelp, sea lettuce (*Ulva lactuca*), dulse (*Palmaria palmate*), and purple layer (*Porphyra,* e.g., used in nori). Regarding microalgae, some species of *Chlorella* have for many years been popular in Japan and Korea as a daily used nutritional supplement in the same way many westerners ingest a fish oil capsule for the omega oil content (the fish get the omega oil by eating algae at sea). Also, the green Spirulina type (*Arthrospira platensis*) is grown commercially in open ponds (photosynthetically) for use as a nutritional supplement or incorporated in small quantities in smoothies and other drinks (normally less than 0.5% w/w).

The problem with off-taste of *Chlorella vulgaris* is e.g. addressed in WO 2021/219562, which relates to a two-step heat treatment, which reduces the off-taste in the end-product.

WO 2020/105001 A1 (Spicer Consulting Limited, 2020) discloses strains of *Chlorella vulgaris* with a reduced chlorophyll content (reduced down to 0.001 mg/g algae) as means to reduce off-taste problems, unpleasant aroma and undesirable colour problems with end-products comprising *Chlorella vulgaris* biomass.

WO 2021/240232 A1 (Algenuity Holdings Ltd) also discloses strains of *Chlorella vulgaris* with a reduced chlorophyll content (reduced down to 0.001 mg/g algae) as means to reduce off-taste problems, unpleasant aroma and undesirable colour problems with end-products comprising *Chlorella vulgaris* biomass. Specific gene mutations in the *Chlorella vulgaris* genome, which are related to the low chlorophyll content are also disclosed.

Touching upon the content of omega oils in algae, algal oil with high docosahexanoic acid (DHA) content is seen used as an ingredient in various products for people with special dietary needs. DHA is polyunsaturated oil with well-researched anti-inflammatory properties. However, DHA is not suitable for other than raw foods because it oxidizes with heat treatment. DHA is furthermore unstable when exposed to oxygen, even at room temperature, causing a fishy taste and unpleasant aroma.

There remains a strong need for microalgae to produce tasty and nutritious foodstuff, cheaply and efficiently and at large scale.

### SUMMARY OF THE INVENTION

The present inventors have developed strains of *Chlorella vulgaris* which have exceptionally low (as low as <0.000001 mg/g dry cell weight) or undetectable chlorophyll content. Biomass derived from this strain can advantageously be used in foodstuff for human consumption, since the low chlorophyll content ensures a lack of the dark colour and unpleasant taste and smell caused by chlorophyll.

So, in a first aspect the present invention relates to a modified strain of *Chlorella vulgaris* having a chlorophyll B content of <0.00015 mg/g dry cell weight. Related to the first aspect is also provided a modified strain of *Chlorella vulgaris,* which contains no detectable chlorophyll B when analysing for chlorophyll content with the method set forth in Example 2 and/or which contains no detectable chlorophyll A when analysing for chlorophyll content as described in Example 2.

In a 2^{nd} aspect, the present invention provides a dried product comprising or consisting essentially of biomass of the modified strain of the 1^{st} aspect of the invention and any embodiments thereof disclosed herein.

In a 3^{rd} aspect, the present invention provides a composition comprising biomass derived from the modified strain of *Chlorella vulgaris* of the 1^{st} aspect of the invention and any embodiments thereof disclosed herein, and further comprising one further ingredient in the form of a food; a feed; a food or feed additive; or a nutritionally acceptable stabilizer, carrier, diluent, or excipient.

In a 4^{th} aspect the present invention provides use of the strain according of the 1^{st} aspect of the invention and any embodiments thereof disclosed herein, of the dried product of the 2^{nd} aspect of the invention and any embodiments thereof disclosed herein, or the composition of the 3^{rd} aspect of the invention and any embodiments thereof disclosed herein, as a foodstuff, food ingredient, dietary supplement, cosmetic product, or a personal care product, or as an ingredient in a foodstuff, food ingredient, dietary supplement, cosmetic product, or a personal care product.

Finally, in a 5^{th} aspect the present invention relates to method of producing a modified strain of *Chloralla vulgaris* of the first aspect of the invention and any embodiments thereof disclosed herein, comprising the steps of i) inoculating and culturing samples of a chlorophyll-containing *Chloralla vulgaris* strain, such as a wild-type strain, for a period of time, preferably in a liquid growth medium, ii) transferring a sample cultured in step ii) to a solid growth medium and subjecting it to mutagenesis, preferably via irradiation with ultraviolet light, iii) incubating the sample on the growth medium from step ii) for a period of time, iv) selecting non-green colonies, and optionally culturing the colonies selected to exclude colonies showing signs of phenotype reversion, and v) selecting the modified strain from the colonies of step iv as being one that analytically exhibits a lowered or undetectable chlorophyll content as defined above in respect of the 1^{st} aspect of the invention as well as embodiments thereof disclosed herein.

### LEGENDS TO THE FIGURE

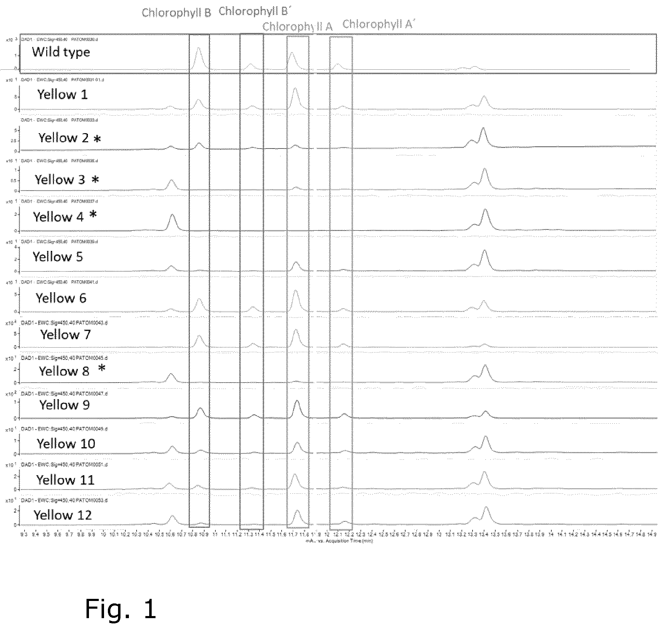

Fig. 1. UV chromatograms (450 nm) of each of the microalgae strains; strains marked with "*" indicate no chlorophyll detection.

### DETAILED DISCLOSURE OF THE INVENTION

### Definitions

*Chlorella vulgaris* is a green unicellular eukaryotic microalga belonging to the genus *Chlorella,* originally discovered in 1890 by Martinus Willem Beijerinck as the first microalga with a welldefined nucleus. It is recognized for a high protein content and has for decades been considered a new food source. The nuclear genome of *C*. *vulgaris* 211/11P has been analysed and annotated (Cecchin M. et al. 2019, The Plant Journal 100: 1289-1305), which has revealed a total of 10,724 genes coding for 11,082 transcripts with an average length of 3062 base pairs and 8.12 exons per average gene.

A "modified strain" denotes a strain of C. *vulgaris,* which has been mutated into a stably growing, viable strain, which exhibits the low chlorophyll content disclosed for a number of strains herein after multiple cell generations in culture. For the most preferred strains, the genes responsible for the expression by C. *vulgaris* of chlorophyll have been "knocked out", for instance because the mutations introduced have disrupted the coding sequences for one or more of the enzymes responsible for chlorophyll synthesis and/or disrupted necessary expression control elements for these enzymes. The expression "modified strain" is used interchangeably with the expression "mutated strain" herein.

A "derivative" of a specifically identified *C*. *vulgaris* strain is a strain, which maintains the genomic characteristics of the specifically identified strain, which are responsible for the chlorophyll phenotype. For instance, the genomic differences between the specifically identified strain DTU-CV-138/K (cf. below) provided in the present disclosure and *C*. *vulgaris* 211/11P, which give rise to the chlorophyll deficient phenotype in DTU-CV-138/K, constitute a genomic fingerprint; so a derivative of DTU-CV-138/K will be a strain, which shares this genomic fingerprint, i.e. the mutations in the same expressed genes that are responsible for the deficient chlorophyll expression in DTU-CV-138/K. It is routine for a skilled person to fully sequence the genomic DNA of a microorganism and thereby identify differences (mutations) relative to a reference genome and it is likewise routine to determine whether genomic mutations are silent or have effects on expression products. In particular, and as reported in WO 2021/240232, mutations in genes responsible for tetrapyrrole biosynthesis pathways, chlorophyll biosynthesis pathways, or carotenoid biosynthesis pathways can be responsible for altered phenotypes of Chlorella vulgaris, which have low chlorophyll content. In particular, non-silent mutations in genes encoding any one of magnesium chelatase subunit I, O-methyltransferase, magnesium-protoporphyrin O-methyltransferase, 15-cis-phytoene desaturase, or zeta-carotene desaturase, can have profound effects on the chlorophyll phenotype. So, if mutations in the presently identified strains are present in any of the *Chlorella vulgaris* strains of the present invention and in any of these genes, then strains that contain the same mutations in the same genes are considered to be derivatives of the presently identified strains.

### Specific embodiments of the invention

### 1^{st} aspect of the invention

As indicated above, the modified strains of *C*. *vulgaris* disclosed herein all exhibit a considerably lower chlorophyll content than the strains disclosed in WO 2020/105001 and WO 2021/240232. It is believed that the present inventors are the first to provide viable *C*. *vulgaris* strains that exhibit no detectable chlorophyll and even the first to provide *C*. *vulgaris* having a chlorophyll B content of <0.00015 mg/g dry cell weight; notably, Figs. 5-10 disclosed in WO 2020/105001 all show a detectable amount of chlorophyll in all tested strains as determined by absorbance measurements at 647, 664, and 750 nm; the same Figs. 5-10 are disclosed in WO 2021/240232. In contrast, and as shown in the present examples, UV radiation mutated strains of the present invention have been shown to exhibit no detectable chlorophyll B and/or no detectable chlorophyll A, thus evidencing that it is possible to obtain and maintain such strains that have significantly lower amounts of chlorophyll than any available prior art strains, at least when cultured under the conditions disclosed in Example 1.

The modified strains of the present invention preferably are those, which further (*i.e*. in addition to their low chlorophyll B content) also exhibit a chlorophyll A content <0.00015 mg/g dry cell weight. It is in particular preferred that the strains exhibit a total chlorophyll content of <0.00015 mg/g dry cell weight. However, as demonstrated in the examples reported herein, the chlorophyll B content can be much lower, such as <0.00001 mg/g dry cell weight, preferably <0.000001 mg/g dry cell weight; the same is true for the chlorophyll A content, which can be <0.00001, preferably <0.000001 mg/g dry cell weight. And, as demonstrated in the examples, strains of *C*. *vulgaris* have been provided where the total chlorophyll content is <0.00001, preferably <0.000001 mg/g dry cell weight; such strains are in especially preferred.

It is believed that at least 4 of the strains disclosed in the examples are completely free of chlorophyll: or, put differently, the invention provides strains of *C*. *vulgaris,* which are exemplary of preferred embodiments of the 1^{st} aspect of the invention, and which contain no detectable chlorophyll B when analysing for chlorophyll content with the method set forth in Example 2. Likewise, and with support in the examples, the invention provides modified strains of *Chloralla vulgaris,* which contain no detectable chlorophyll A when analysing for chlorophyll content as described in Example 2. The preferred modified strains of the present invention contain no detectable chlorophyll when analysing for chlorophyll content as described in Example 2.

As part of the work leading to the provision of the present invention, it was found that some mutated strains under standard culture conditions could revert to a phenotype containing higher amounts of chlorophyll than those referenced above. On the other hand, prolonged culture of the mutated *C*. *vulgaris* strains revealed that some had been mutated to provide for strains which that stably maintained the reduced/undetectable chlorophyll content after multiple cell cycles in culture. Such stable strains are preferred embodiments of the 1^{st} aspect of the invention.

Since the preferred strains of the invention appear to lack chlorophyll altogether, their colours differ significantly from the natural dark green colour of *C*. *vulgaris* wild-type strains, meaning that a strain of the 1^{st} aspect of the invention typically in "non-green", *i.e*. has a colour selected from white, cream, pale yellow, yellow, golden, caramel, orange, and red.

*C. vulgaris* strains are recognized as containing a high protein content; according to Safi et *al.* 2014, the total protein content of the dry weight of *C*. *vulgaris* ranges between 43-58%, depending on growth conditions, meaning that a modified strain of the 1^{st} aspect of the invention has a minimum protein content of at least 15%, such as at least 15, 16, 17, 18. 19, 20, 21, 22, 23, 24, 25, 26, 27, 28, 29, 30, 31, 32, 33, 34 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, or 65% (dry weight w/w) - here the fact that chlorophyll content is reduced is believed to allocate metabolic resources for synthesis of other protein.

The presently preferred modified strain is strain DTU-CV-138/K, deposited by the applicant under the terms of the Budapest Treaty with the Banco Español de Algas (BEA) under accession number BEA_IDA_0076 on 28 October 2021; also preferred is progeny as well as or derivatives of said strain.

Further, modified strains DTU-CV-236/K, DTU-CV-237/K, DTU-CV-242/K have been deposited by the applicant under the terms of the Budapest Treaty with the Banco Español de Algas (BEA); also these strains as well as progeny and derivatives of each of these strains are preferred embodiments of the present invention.

### 2^{nd} aspect of the invention

This aspect relates to a dried product comprising or consisting essentially of biomass of the modified strain according to any one of the preceding claims.

As mentioned under the background section, *C*. *vulgaris* is considered a "superfood", *i.a.* due to its very high content of protein. Hence, any type of product, which currently is marketed and where *C*. *vulgaris* biomass is a constituent, could utilise biomass from the presently disclosed modified strains. Such biomass is conveniently processed into a dried product by methods known per se, cf. Ching-Lung Chen et al. 2015, Drying Technology 33(4): 443-454, doi.org/10.1080/07373937.2014.997881, and according to the present invention made available in the form of a flour, a dried homogenate, pellets, tablets, or agglomerated particles.

This subsequently allows for the admixture of the dried product into a diverse group of products, in particular food products, where protein from other sources is supplemented with or substituted by the dried product having the high protein content, cf. the discussion of compositions *infra.*

### 3^{rd} aspect of the invention

This aspect relates to a composition comprising biomass derived from the modified strain of *Chloralla vulgaris* according of the 1^{st} aspect of the invention as well as embodiments thereof disclosed herein. This composition further comprises at least one further ingredient in the form of a food; a feed; a food or feed additive; or a nutritionally acceptable stabilizer, carrier, diluent, or excipient. The preferred compositions are food compositions, or at least compositions for human ingestion. Nevertheless, the preferred composition is a food or a food ingredient for human consumption, an animal feed or an animal feed ingredient, a cosmetic product or a cosmetic product ingredient, a personal care composition or ingredient thereof, a pharmaceutical composition or an ingredient thereof, a neutraceutical composition for human or animal use of an ingredient thereof, a pasta product, a dressing product, a mayonnaise product, a cake product, a bread product, an energy bar, a milk product, a juice product, a spread, or a smoothie or a food supplement, ingredient or chemical component based on one or more parts of the algae, for example from the cell wall or nucleus.

Such composition of the invention typically comprises 5-30% of dry weight of said strain per dry weight composition, such as about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, and about 30%. However, in certain embodiments the content can be even higher, such as at least 30%, at least 35% and at least 40%.

### 4^{th} aspect of the invention

This aspect relates to practical utilisations of the products of the 1^{st} - 3^{rd} aspects of the invention. In other words, this aspect relates to use of the strain of the 1^{st} aspect of the invention and all embodiments thereof disclosed herein, the dried product of the 2^{nd} aspect of the invention as well as any embodiments thereof disclosed herein, or the composition of the 3^{rd} aspect of the invention as well as any embodiments thereof disclosed herein, as a foodstuff, food ingredient, dietary supplement, cosmetic product, or a personal care product, or as an ingredient in a foodstuff, food ingredient, dietary supplement, cosmetic product, or a personal care product. In other words, the *C*. *vulgaris* biomass of the present invention finds used in any product, where C. *vulgaris* biomass has been previously utilised or contemplated as an ingredient, but as mentioned above, the use of this aspect of the invention is particularly relevant when the products are used in human nutrition.

### 5^{th} aspect of the invention

This aspect generally relates to production of a modified strain of the 1^{st} aspect of the invention and comprise the steps of i) inoculating and culturing samples of a chlorophyll-containing *Chloralla vulgaris* strain, such as a wild-type strain, for a period of time, preferably in a liquid growth medium, ii) transferring a sample cultured in step ii) to a solid growth medium and subjecting it to mutagenesis, preferably via irradiation with ultraviolet light, iii) incubating the sample on the growth medium from step ii) for a period of time, iv) selecting non-green colonies, and optionally culturing the colonies selected to exclude colonies showing signs of phenotype reversion, and v) selecting the modified strain from the colonies of step iv as being one that analytically exhibits the desired lowered or undetectable chlorophyll content.

As shown in the examples, mutagenesis via UV radiation is one effective means, but any mutagenesis method which is not lethal to the *C*. *vulgaris* strain is useful and can hence involve other types of ionzing radiation (X-rays, γ-rays, β-rays) as well as chemical mutagenesis such as Methyl methanesulfonate and N-methyl-N'-nitro-N-nitrosoguanidine.

The period of time in step i) is not of high essence but is typically selected to be sufficient to be able to transfer a sample of 10⁷ cells which are in mid-exponential growth phase to step ii).

### Microalgae fermentation

In the following is provided details for a suitable fermentation and harvesting/drying procedure of the microalgae of the present invention, and thus constitute steps applicable for several of the aspects disclosed above:
1) During fermentation all equipment, air, media etc. must be sterilized, and kept sterile.
2) The temperature of the bioreactor is kept at 28°C, aeriation set to 1 l air per minute per 1 l vessel volume. A 300 l vessel would e.g. require 600 l air per minute.
3) High agitation is employed, preferably using a Rushton impeller.
4) Acidity is continuously adjusted with KOH and H₃PO₄, to maintain pH 6.2.
5) The following media are used to sustain growth of the microalgae.

| **Compound** | **Amount in g per 1 l media** |
|---|---|
| KH₂PO₄ | 1 |
| K₂HPO₄ | 1 |
| Yeast extract | 4 |
| Glucose | 30 |

6) The fermentation can be run in batch mode, although continuous mode can also be used.
7) Before fermentation is started, the vessel is sterilized with the media and its constituent added, except for glucose.
8) Sterile glucose is consciously added, to maintain a glucose level at 30 g/l during fermentation.
9) When the cell concentration reaches 50 g DCW/l or higher (DCW = Dry Cell Weight), the broth is centrifuged, and then spray dried, to achieve the final product.

### EXAMPLE 1

### Preparation of chlorophyll deficient Chlorella vulgaris strains

### Strains and media

A wild type isolate from the species *Chlorella vulgaris* was used as the subject of mutagenesis experiments, using UVC irradiation. All the mutant *C*. *vulgaris* strains subsequently obtained by this process are hence derived from this wild-type strain.

Strain maintenance, UVC irradiation, and mutant growth and selection were performed using solid medium plates containing KH₂PO₄ (1 g/l), K₂HPO₄ (1 g/l), yeast extract (Bacto) (4 g/l), agar (Bacto) (15 g/l), and glucose (30 g/l). The pH of the medium was adjusted to 6.3 using 2 M H₂SO₄ prior to sterilization in an autoclave at 121°C for 20 minutes. The carbon source (glucose) was sterilized separately from the rest of the components. Deionized water was used for the preparation of all solutions.

For liquid cultivation, a modified recipe of Gamborg's B5 medium was used. The microalgae strains were grown using a 1:4 dilution of the modified B5 recipe, to which a carbon source was added for heterotrophic growth. The undiluted modified B5 medium contained KH₂PO₄ (1769.12 mg/l), K₂HPO₄ (749.06 mg/l), MgSO₄·7H₂O (250 mg/l), CaCl₂·2H₂O (150 mg/l), NH₃ (0.15 g/l), biotin (0.1 mg/l), thiamine hydrochloride (2 mg/l), pyridoxine (1 mg/l), and the following trace elements: Na₂-EDTA (37.3 mg/l), MnSO₄·H₂O (10 mg/l), KI (0.75 mg/l), CuSO₄·5H₂O (0.025 mg/l), FeSO₄·7H₂O (27.8 mg/l), CoCl₂·6H₂O (0.025 mg/l), ZnSO₄·7H₂O (2 mg/l), Na₂MoO₄·2H₂O (0.25 mg/l), and H₃BO₃ (3 mg/l). The pH of the medium was adjusted to 6.3 using 2 M H₂SO₄, prior to being sterilized by filtration. The 1:4 dilution of the medium was supplemented with 20 g/l of glucose for heterotrophic growth. Deionized water was used for the preparation of all the solutions.

### UV-C mutagenesis procedure and mutant screening

Using a 10 µL inoculation loop, a scoop of the wild type strain biomass (approximately 0.02 g) was taken from a solid medium plate and grown under light (12 hours light/darkness cycles) at 22°C in 12 ml culture tubes containing 5 ml of liquid medium. The tubes were agitated at 150 rpm, and the cap of the tube remained loose to facilitate gas exchange. After 2 days, a volume of the culture containing 1·10⁷ cells in mid-exponential growth phase (counted using a hemocytometer) was added onto solid medium plates, spread over the whole surface using a sterile spatula, and radiated using a UVC lamp (Mineralight^{®} UVSL-25, Ultra-Violect Products, Inc., USA) at a wavelength of 254 nm. The distance between the lamp and the medium plates was 15 cm (Anthony et al., 2015; Sarayloo et al., 2017) and the exposure time was 30 minutes. After the UV treatment, the plates were sealed using gas permeable sealing film, wrapped in aluminum foil to avoid light and prevent light-induced repair, and finally incubated at 22°C for one week.

After incubation, plates were visually inspected for light colored colonies, which were isolated using sterile inoculation loops, plated onto solid medium plates, sealed using permeable sealing film, and grown under light (12 hours light/darkness cycles) at 22°C.

After the mutagenesis, the light colored colonies were grown (as described herein) for several cell cycles in order to de-select colonies that reverted to a green (*i.e*. chlorophyll-containing) phenotype. Only colonies that maintained the light color were subsequently selected for further analysis, as described in following Example 2.

### EXAMPLE 2

### Chlorophyll detection and strain selection

### Pigment extraction from microalgae

For each strain of microalgae obtained in Example 1, the following pigment extraction procedure was carried out:
1. Microalgae were harvested and freeze-dried 48h in the dark.
2. The freeze-dried microalgae where grinded into a powder with liquid nitrogen.
3. 20 mg of freeze-dried microalgae were mixed with 3 ml of Ethanol/Hexane (2:1, v/v) containing 0.1% (w/v) butylated hydroxytoluene (antioxidant) in a glass tube.
4. The mixture was ultrasonicated in the dark for 20 min.
5. 2 ml H₂O and 4 ml of Hexane were added.
6. The mixture from step 5 was vortexed thoroughly and centrifuged at 4°C and 1,000 × g for 5 min.
7. 4 ml of the hexane layer (upper layer) was transferred to a separate tube and evaporated under N₂ at 25±2 °C in the dark.
8. 4 ml Hexane was added to the tube, and steps 6 and 7 were repeated (Twice)
9. The mixture from step 8 was reconstituted in 500 µl methyl tertiary butyl ether + acetonitrile (MTBE + ACN) (75+25, v + v)
10. The reconstituted mixture from step 9 was stored at -80°C until injection.

### Chlorophyll detection

Qualitative analysis of chlorophyll was performed on an ultra-high performance liquid chromatography diode array detection quadrupole time of flight mass spectrometry (UHPLC-DAD-QTOF-MS). The conditions were as follows:
1. Mobile phase A was a mixture of ACN + methanol (MeOH) + MTBE (70+20+10, v/v/v) and mobile phase B was 10 mM ammonium acetate in water.
2. Agilent Poroshell 120 phenyl-hexyl column (150mm × 2.1mm, 1.9 µm).
3. Column temperature: 45°C; flow rate: 0.5 ml/min.
4. Mobile phase gradient: 65% mobile phase B at 0 -1 min increase to 100% B at 16 min, 65% B between 18 - 20 min.
5. DAD detector 450 nm.
6. Mass spectrometry condition: the MS was operated in positive ionization mode (ESI+), centroid data was recorded in the 50 -1700 m/z mass range in both MS mode and MS/MS mode. Acquisition rate was 10 spectras/s; data dependent acquisition (DDA) MS/MS data was recorded with fixed collision energies of 10 eV, 20 eV, and 40 eV with maximum 3 precursor ions per cycle.

To quantify the chlorophyll content, a standard curve is prepared by correlating known concentrations of chlorophyll with the corresponding signals in the MS. Also, the efficacy of the above extraction process is checked to ensure that all chlorophyll from a dried algae preparation is found in one single HPLC fraction of the extract. Hence, the signal obtained on the samples tested herein can be translated into a chlorophyll concentration (w/v) and since the chlorophyll is the complete amount in a known amount of dried sample, a dry weight concentration (w/w) in the original algae sample can be determined.

### Results and discussion

Chlorophyll A and Chlorophyll B was initially detected by UV absorption at 450 nm (see Fig. 1). Strains "yellow 4" and "yellow 8" exhibited no UV absorption at 450 nm and therefore lack both chlorophyll A and B.

Strains "Yellow 2" and "Yellow 3" exhibited UV absorptions at 450 nm. Therefore, the Extracted Ion Chromatograms (EIC) of chlorophyll A (m/z 893.5427, [M+H]⁺) and chlorophyll B (m/z 907.5214, [M+H]⁺) were investigated in more detail; see Table 1 below. Strains "Yellow 2" and "Yellow 3" exhibited integratable areas of these two m/z, but no MS2 spectrums were triggered. Therefore, the chlorophyll A and B content in these 2 strains was below the detection limit of the instrument, which is in the picomolar to femtomolar range. This thus translates into a mass concentration < 0.000001 mg/g dry cell weight.

**Table 1**

| Retention time and Area of Extracted Ion Chromatograms (EIC) of chlorophyll A and B. | | | | | | | | |
|---|---|---|---|---|---|---|---|---|
| Strain | **Chlorophyll A** | | **Chlorophyll A** | | **Chlorophyll B** | | **Chlorophyll B** | |
| | RT | Area | RT | Area | RT | Area | RT | Area |
| Wild type | 12.0 | 3,605,114 | 12.4 | 2,202,750 | 11.2 | 1,648,041 | 11.6 | 881,761 |
| Yellow 1 | 11.8 | 1,083,460 | 12.22 | 138,116 | 10.9 | 114,480 | 11.4 | 36,432 |
| Yellow 2* | 11.8 | *7,312.04* | 12.22 | *1,088.77* | 10.9 | *1,674.58* | 11.4 | |
| Yellow 3* | 11.8 | *11,015.08* | 12.22 | *1,495.53* | | | | |
| Yellow 4* | | | | | | | | |
| Yellow 5 | 11.8 | 228,306 | 12.22 | 21,459.5 | 10.9 | | 11.4 | |
| Yellow 6 | 11.8 | 626,162 | 12.22 | 64,011.6 | 10.9 | 92,194 | 11.4 | 25,933 |
| Yellow 7 | 11.8 | 2,585,625 | 12.22 | 1,075,355 | 10.9 | 910,746 | 11.4 | 364,838 |
| Yellow 8* | | | | | | | | |
| Yellow 9 | 11.8 | 1,145,669 | 12.22 | 261,143 | 10.9 | 191,765 | 11.4 | 52,867 |
| Yellow 10 | 11.8 | 125,138 | 12.22 | 15,230.6 | | | | |
| Yellow 11 | 11.8 | 217,337 | 12.22 | 11,473.1 | | | | |
| Yellow 12 | 11.8 | 256,349 | 12.22 | 31,551.3 | | | | |
| Extraction | | | | | | | | |
| blank | | | | | | | | |

| | | | | | | | | |
|---|---|---|---|---|---|---|---|---|
| *) Strain "Yellow 4" is identical to strain DTU-CV-138/K, deposited by the applicant under the terms of the Budapest Treaty with the Banco Español de Algas (BEA) under accession number BEA_IDA_0076 on 28 October 2021. Strains "Yellow 2", "Yellow 3" and "Yellow 8" are identical to strains DTU-CV-236/K, DTU-CV-237/K, and DTU-CV-242/K, respectively, all deposited by the applicant under the terms of the Budapest Treaty with the Banco Español de Algas (BEA). | | | | | | | | |

In conclusion, Strains "Yellow 2", "Yellow 3", "Yellow 4" and "Yellow 8" contain neither chlorophyll A nor B. Strains "Yellow 5", "Yellow 10", "Yellow 11" and "Yellow 12" contain only chlorophyll A. Strains "Yellow 1", "Yellow 6", "Yellow 7" and "Yellow 9" contain both Chlorophyll A and B, but all mutants have low amounts of both Chlorophyll A and B compared to the wild type.

### LIST OF REFERENCES

1. Anthony, J. et al. (2015). Marine Biotechnology, 17(1), 66-80. https://doi.org/10.1007/s10126-014-9597-5
2. Sarayloo, E. et al. Algal Research, 28(November), 244-252. https://doi.org/10.1016/j.algal.2017.11.009

BUDAPEST TREATY ON THE INTERNATIONAL
RECOGNITION OF THE DEPOSIT OF MICROORGANISMS
FOR THE PURPOSES OF PATENT PROCEDURE
STATEMENT IN THE CASE OF AN ORIGINAL DEPOSIT

INTERNATIONAL FORM

TO

Name: Biotrino ApS

Address: Fruebjergvej 3,
2100 Copenhagen
Denmark

RECEIPT IN THE CASE OF AN ORIGINAL DEPOSIT
issued pursuant to Rule 7.1 by the
SPANISH BANK OF ALGAE (BEA)
Identified at the bottom of this page

I. IDENTIFICATION OF THE MICROORGANISM

Identification reference given by the DEPOSITOR: | Accession number given by the BEA:

DTU-CV-138/K | BEA_IDA_0076

II. SCIENTIFIC DESCRIPTION AND/OR PROPOSED TAXONOMIC DESIGNATION

The microorganism identified under I above was accompanied by:

❐ a scientific description
X a proposed taxonomic designation

(Mark with a cross where applicable)

III. RECEIPT AND ACCEPTANCE

BEA accepts the microorganisms identified under I above, which was received by it on 28th of October 2021.

(date of the original deposit 1 )

IV. RECEIPT OF REQUEST FOR CONVERSION

The microorganism identified under I above was received by this International Depositary Authority on (not applicable) (date of the original deposit) and a request
to convert the original deposit to a deposit under de Budapest Treaty was received by it on (not applicable) (date of receipt of request for conversion)

V. INTERNATIONAL DEPOSITARY AUTHORITY

Banco Español de Algas (BEA) | Date: 28th of October 2021

Universidad de Las Palmas de GC | Curator of BEA culture collection | Legal representative of the University of Las Palmas de Gran Canaria
Muelle de Taliarte, s/n
35214 Telde – Las Palmas
España

Dr. Antera Martel Quintana | Dr. María Soledad Izquierdo López
Vice-rector for Research and Transfer

1 Where Rule 6.4(d) applies, such date is the date on which the status of international depositary authority was acquired

Form BP/4 (sole page)

## Claims

1. A modified strain of *Chlorella vulgaris* having a chlorophyll B content of <0.00015 mg/g dry cell weight.

2. The modified strain according to claim 1, which further has a chlorophyll A content <0.00015 mg/g dry cell weight.

3. The modified strain according to claim 1 or 2, which has a total chlorophyll content of <0.00015 mg/g dry cell weight.

4. The modified strain according to any one of the preceding claims, wherein the chlorophyll B content is <0.00001, preferably <0.000001 mg/g dry cell weight; and/or wherein the chlorophyll A content is <0.00001 mg/g dry cell weight, preferably <0.000001 mg/g dry cell weight; and/or wherein the total chlorophyll content is <0.00001 mg/g dry cell weight, preferably <0.000001 mg/g dry cell weight.

5. The modified strain according to any one of the preceding claims, which contains no detectable chlorophyll B when analysing for chlorophyll content with the method set forth in Example 2; and/or which contains no detectable chlorophyll A when analysing for chlorophyll content using ultra-high performance liquid chromatography diode array detection quadrupole time of flight mass spectrometry, preferably as described in Example 2; and/or which contains no detectable chlorophyll when analysing for chlorophyll content using ultra-high performance liquid chromatography diode array detection quadrupole time of flight mass spectrometry, preferably as described in Example 2.

6. The modified strain according to any one of the preceding claims, which stably maintains the reduced/undetectable chlorophyll content after multiple cell cycles in culture.

7. The modified strain according to any one of the preceding claims, which has a minimum protein content of at least 15% dry weight w/w, such as at least 15, 16, 17, 18, 19, 20, 21, 22, 23, 24, 25, 26, 27, 28, 29, 30, 31, 32, 33, 34 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, or 65%.

8. The modified strain according to any one of the preceding claims, which is strain DTU-CV-138/K, deposited under the terms of the Budapest Treaty with the Banco Español de Algas (BEA) under accession number BEA_IDA_0076 on 28 October 2021 or which is a progeny or derivative of strain DTU-CV-138/K.

9. A dried product comprising or consisting essentially of biomass of the modified strain according to any one of the preceding claims.

10. The dried product according to claim 9, which is in the form of a flour, a dried homogenate, pellets, tablets, or agglomerated particles.

11. A composition comprising biomass derived from the modified strain of *Chlorella vulgaris* according to any one of claims 1-8, and comprising one further ingredient in the form of a food; a feed; a food or feed additive; or a nutritionally acceptable stabilizer, carrier, diluent, or excipient.

12. The composition according to claim 11, wherein the composition is a food or a food ingredient for human consumption, an animal feed or an animal feed ingredient, a cosmetic product or a cosmetic product ingredient, a personal care composition or ingredient thereof, a pharmaceutical composition or an ingredient thereof, a neutraceutical composition for human or animal use or an ingredient thereof, a pasta product, a dressing product, a mayonnaise product, a cake product, a bread product, an energy bar, a milk product, a juice product, a spread, or a smoothie, or a food supplement, ingredient or chemical component based on one or more parts of the algae, for example from the cell wall or nucleus.

13. The composition according to any one of claims 11-12, which comprises 5-30% of dry weight of said strain per dry weight composition, such as about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, and about 30%.

14. Use of the strain according any one of claims 1-8, the dried product of claim 9 or 10, or the composition of any one of claims 11-13, as a foodstuff, food ingredient, dietary supplement, cosmetic product, or a personal care product, or as an ingredient in a foodstuff, food ingredient, dietary supplement, cosmetic product, or a personal care product, wherein the composition preferably is used in human nutrition.

15. A method of producing a modified strain of *Chlorella vulgaris* according to any one of claims 1-8, comprising the steps of
i) inoculating and culturing samples of a chlorophyll-containing *Chlorella vulgaris* strain, such as a wild-type strain, for a period of time, preferably in a liquid growth medium,
ii) transferring a sample cultured in step ii) to a solid growth medium and subjecting it to mutagenesis, preferably via irradiation with ultraviolet light,
iii) incubating the sample on the growth medium from step ii) for a period of time,
iv) selecting non-green colonies, and optionally culturing the colonies selected to exclude colonies showing signs of phenotype reversion, and
v) selecting the modified strain from the colonies of step iv as being one that analytically exhibits a lowered or undetectable chlorophyll content as defined in any one of claims 1-8, wherein the period of time in step i) preferably is sufficient to be able to transfer a sample of 10⁷ cells which is in mid exponential growth phase to step ii).

## Patentansprüche

1. Modifizierter Stamm von *Chlorella vulgaris,* der einen Chlorophyll B-Gehalt von <0,00015 mg/g Trockenzellengewicht aufweist.

2. Modifizierter Stamm nach Anspruch 1, der ferner einen Chlorophyll A-Gehalt von <0,00015 mg/g Trockenzellengewicht aufweist.

3. Modifizierter Stamm nach Anspruch 1 oder 2, der einen gesamten Chlorophyllgehalt von <0,00015 mg/g Trockenzellengewicht aufweist.

4. Modifizierter Stamm nach einem der vorhergehenden Ansprüche, wobei der Chlorophyll B-Gehalt <0,00001, bevorzugt <0,000001 mg/g Trockenzellengewicht beträgt; und/oder wobei der Chlorophyll A-Gehalt <0,00001 mg/g Trockenzellengewicht, bevorzugt <0,000001 mg/g Trockenzellengewicht beträgt; und wobei der gesamte Chlorophyllgehalt <0,00001 mg/g Trockenzellengewicht, bevorzugt <0,000001 mg/g Trockenzellengewicht beträgt

5. Modifizierter Stamm nach einem der vorhergehenden Ansprüche, der kein erfassbares Chlorophyll B enthält, wenn er bezüglich des Chlorophyllgehalts mit dem Verfahren analysiert wird, das in Beispiel 2 aufgeführt ist; und/oder der kein erfassbares Chlorophyll A enthält, wenn er bezüglich des Chlorophyllgehalts unter Anwendung von Ultrahochleistungs-Flüssigchromatographie-Diodenanordnungserfassungs-Quadrupolflugzeitmassenspektrometrie, bevorzugt wie in Beispiel 2 beschrieben, analysiert wird; und/oder der kein erfassbares Chlorophyll enthält, wenn er bezüglich des Chlorophyllgehalts unter Anwendung von Ultrahochleistungs-Flüssigchromatographie-Diodenanordnungserfassungs-Quadrupolflugzeitmassenspektrometrie, bevorzugt wie in Beispiel 2 beschrieben, analysiert wird.

6. Modifizierter Stamm nach einem der vorhergehenden Ansprüche, der den reduzierten/nicht erfassbaren Chlorophyllgehalt nach mehreren Zellzyklen in Kultur beständig beibehält.

7. Modifizierter Stamm nach einem der vorhergehenden Ansprüche, der einen Mindestproteingehalt von mindestens 15 Gew.-% Trockengewicht, wie beispielsweise mindestens 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64 oder 65 Gew.-% aufweist.

8. Modifizierter Stamm nach einem der vorhergehenden Ansprüche, der der Stamm DTU-CV-138/K ist, der unter den Bedingungen des Budapester Übereinkommens mit Banco Español de Algas (BEA) unter der Zugangsnummer BEA_IDA_0076 am 28. Oktober 2021 hinterlegt worden ist oder der ein Abkömmling oder Derivat von Stamm DTU-CV-138/K ist.

9. Getrocknetes Produkt umfassend oder bestehend im Wesentlichen aus Biomasse des modifizierten Stamms nach einem der vorhergehenden Ansprüche.

10. Getrocknetes Produkt nach Anspruch 9, das in Form eines Mehls, eines getrockneten Homogenats, von Pellets, Tabletten oder angehäuften Partikeln vorliegt.

11. Zusammensetzung umfassend Biomasse, die von dem modifizierten Stamm von *Chlorella vulgaris* nach einem der Ansprüche 1-8 abgeleitet ist und einen weiteren Bestandteil in Form eines Nahrungsmittels, eines Futters, eines Nahrungsmittel- oder Futterzusatzmittels oder eines ernährungsmäßig akzeptablen Stabilisators, Trägers, Verdünnungsmittels oder Trägerstoffs umfasst.

12. Zusammensetzung nach Anspruch 11, wobei die Zusammensetzung ein Nahrungsmittel oder ein Nahrungsmittelbestandteil für den menschlichen Verzehr, ein Tierfutter oder ein Tierfutterbestandteil, ein kosmetisches Produkt oder ein Bestandteil eines kosmetischen Produkts, eine Körperpflegezusammensetzung oder Bestandteil davon, eine pharmazeutische Zusammensetzung oder ein Bestandteil davon, eine Nutrazeutikumzusammensetzung zum menschlichen oder tierischen Verbrauch oder ein Bestandteil davon, ein Teigwarenprodukt, ein Saucenprodukt, ein Mayonnaiseprodukt, ein Kuchenprodukt, ein Brotprodukt, ein Energieriegel, ein Milchprodukt, ein Saftprodukt, ein Brotaufstrich oder ein Smoothie oder ein Nahrungsergänzungsmittel, -bestandteil oder eine chemische Komponente auf der Basis eines oder mehrere Teile der Alge, beispielsweise aus der Zellwand oder dem Zellkern ist.

13. Zusammensetzung nach einem der Ansprüche 11-12, die 5-30 % Trockengewicht des Stamms pro Trockengewichtzusammensetzung, wie beispielsweise etwa 5 %, etwa 6 %, etwa 7 %, etwa 8 %, etwa 9 %, etwa 10 %, etwa 11 %, etwa 12 %, etwa 13 %, etwa 14 %, etwa 15 %, etwa 16 %, etwa 17 %, etwa 18 %, etwa 19 %, etwa 20 %, etwa 21 %, etwa 22 %, etwa 23 %, etwa 24 %, etwa 25 %, etwa 26 %, etwa 27 %, etwa 28 %, etwa 29 % und etwa 30 % umfasst.

14. Verwendung des Stamms nach einem der Ansprüche 1-8, des getrockneten Produkts nach Anspruch 9 oder 10 oder der Zusammensetzung nach einem der Ansprüche 11-13 als Nahrungsmittel, Nahrungsmittelbestandteil, Nahrungsergänzungsmittel, kosmetisches Produkt oder Körperpflegeprodukt oder als Bestandteil eines Nahrungsmittels, Nahrungsmittelbestandteils, Nahrungsergänzungsmittels, kosmetischen Produkts oder eines Körperpflegeprodukts, wobei die Zusammensetzung bevorzugt zur menschlichen Ernährung verwendet wird.

15. Verfahren zur Herstellung eines modifizierten Stamms von *Chlorella vulgaris* nach einem der Ansprüche 1-8, umfassend die Schritte des
i) Impfens und Züchtens von Proben eines chlorophyllhaltigen *Chlorella vulgaris*-Stamms, wie beispielsweise eines Wildtypstamms, für eine Zeitspanne, bevorzugt in einem flüssigen Wachstumsmedium,
ii) Überführens einer in Schritt ii) gezüchteten Probe in ein festes Wachstumsmedium und Unterwerfens desselben einer Mutagenese, bevorzugt über Bestrahlung mit Ultraviolettlicht,
iii) Inkubierens der Probe auf dem Wachstumsmedium aus Schritt ii) für eine Zeitspanne,
iv) Wählens nichtgrüner Kolonien und wahlweise Züchtens der Kolonien, die ausgewählt sind, um Kolonien auszuschließen, die Anzeichen von Phänotypreversion aufweisen und
v) Auswählens des modifizierten Stamms aus den Kolonien von Schritt iv) als einer, der analytisch einen reduzierten oder nicht erfassbaren Chlorophyllgehalt, wie in einem der Ansprüche 1-8 definiert, aufweist,
wobei die Zeitspanne in Schritt i) bevorzugt ausreicht, in der Lage zu sein, eine Probe von 10⁷ Zellen, die die mittlere exponentielle Wachstumsphase von Schritt ii) ist, zu überführen.

## Revendications

1. Souche modifiée de Chlorella vulgaris ayant une teneur en chlorophylle B inférieure à 0,00015 mg/g de poids sec cellulaire.

2. Souche modifiée selon la revendication 1, qui présente en outre une teneur en chlorophylle A inférieure à 0,00015 mg/g de poids sec cellulaire.

3. Souche modifiée selon la revendication 1 ou la revendication 2, qui a une teneur totale en chlorophylle inférieure à 0,00015 mg/g de poids sec cellulaire.

4. Souche modifiée selon l'une quelconque des revendications précédentes, dans laquelle la teneur en chlorophylle B est inférieure à 0,00001, de préférence inférieure à 0,000001 mg/g de poids cellulaire sec ; et/ou
dans laquelle la teneur en chlorophylle A est inférieure à 0,00001 mg/g de poids cellulaire sec, de préférence inférieure à 0,000001 mg/g de poids sec cellulaire ; et/ou lorsque la teneur totale en chlorophylle est inférieure à 0,00001 mg/g de poids sec cellulaire, de préférence inférieure à 0,000001 mg/g de poids sec cellulaire.

5. Souche modifiée selon l'une quelconque des revendications précédentes, qui ne contient pas de chlorophylle B détectable lors d'une analyse de la teneur en chlorophylle selon la méthode décrite dans l'exemple 2 ; et/ou qui ne contient pas de chlorophylle A détectable lors d'une analyse de la teneur en chlorophylle à l'aide d'une chromatographie liquide haute performance avec détection par réseau de diodes et spectrométrie de masse quadripolaire à temps de vol, de préférence telle que décrite dans l'exemple 2 ; et/ou
qui ne contient pas de chlorophylle détectable lors de l'analyse de la teneur en chlorophylle à l'aide d'une chromatographie liquide ultra haute performance avec détection par barrette de diodes et spectrométrie de masse quadripolaire à temps de vol, de préférence telle que décrite dans l'exemple 2.

6. Souche modifiée selon l'une quelconque des revendications précédentes, qui maintient de manière stable la teneur réduite/indétectable en chlorophylle après plusieurs cycles cellulaires en culture.

7. Souche modifiée selon l'une quelconque des revendications précédentes, qui a une teneur minimale en protéines d'au moins 15 % en poids sur poids sec, telle qu'au moins 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64 ou 65 %.

8. Souche modifiée selon l'une quelconque des revendications précédentes, qui est la souche DTU-CV-138/K, déposée en vertu des dispositions du Traité de Budapest auprès de la Banco Español de Algas (BEA) sous le numéro d'adhésion BEA_IDA_0076 le 28 octobre 2021 ou qui est une descendance ou un dérivé de la souche DTU-CV-138/K.

9. Produit séché comprenant ou constitué essentiellement de biomasse de la souche modifiée selon l'une quelconque des revendications précédentes.

10. Produit séché selon la revendication 9, qui se présente sous la forme d'une farine, d'un homogénat séché, de granulés, de comprimés ou de particules agglomérées.

11. Composition comprenant de la biomasse dérivée de la souche modifiée de Chlorella vulgaris selon l'une quelconque des revendications 1 à 8, et comprenant un ingrédient supplémentaire sous la forme d'un aliment ; un aliment pour animaux ; un additif alimentaire ou pour animaux ; ou un stabilisant, un support, un diluant ou un excipient acceptable sur le plan nutritionnel.

12. Composition selon la revendication 11, dans laquelle la composition est un aliment ou un ingrédient alimentaire destiné à la consommation humaine, un aliment pour animaux ou un ingrédient alimentaire pour animaux, un produit cosmétique ou un ingrédient cosmétique, une composition de soins personnels ou un ingrédient de celle-ci, une composition pharmaceutique ou un ingrédient de celle-ci, une composition neurothérapeutique à usage humain ou animal ou un ingrédient de celle-ci, un produit à base de pâte, un produit de garniture, un produit à base de mayonnaise, un produit à base de gâteau, un produit à base de pain, une barre énergétique, un produit à base de lait, un produit à base de jus, une pâte à tartiner, un smoothie ou un complément alimentaire, un ingrédient ou un composant chimique à base d'une ou plusieurs parties de l'algue, par exemple de la paroi cellulaire ou du noyau.

13. Composition selon l'une quelconque des revendications 11 à 12, qui comprend entre 5 et 30 % du poids sec de ladite souche par composition en poids sec, par exemple environ 5 %, environ 6 %, environ 7 %, environ 8 %, environ 9 %, environ 10 %, environ 11 %, environ 12 %, environ 13 %, environ 14 %, environ 15 %, environ 16 %, environ 17 %, environ 18 %, environ 19 %, environ 20 %, environ 21 %, environ 22 %, environ 23 %, environ 24 %, environ 25 %, environ 26 %, environ 27 %, environ 28 %, environ 29 % et environ 30 %.

14. Utilisation de la souche selon l'une quelconque des revendications 1 à 8, du produit séché selon la revendication 9 ou la revendication 10, ou de la composition selon l'une quelconque des revendications 11 à 13, en tant que denrée alimentaire, ingrédient alimentaire, complément alimentaire, produit cosmétique ou produit de soins personnels, ou en tant qu'ingrédient dans une denrée alimentaire, un ingrédient alimentaire, un complément alimentaire, un produit cosmétique ou un produit de soins personnels, dans laquelle la composition est de préférence utilisée dans l'alimentation humaine.

15. Procédé de production d'une souche modifiée de Chlorella vulgaris selon l'une quelconque des revendications 1 à 8, comprenant les étapes suivantes
i) l'inoculation et la mise en culture d'échantillons d'une souche de Chlorella vulgaris contenant de la chlorophylle, telle qu'une souche de type sauvage, pendant une période de temps, de préférence dans un milieu de culture liquide,
ii) le transfert d'un échantillon cultivé à l'étape ii) vers un milieu de culture solide et son exposition à des mutagènes, de préférence par irradiation aux rayons ultraviolets,
iii) l'incubation de l'échantillon sur le milieu de culture de l'étape ii) pendant une certaine période,
iv) la sélection des colonies non vertes et, éventuellement, la mise en culture des colonies sélectionnées afin d'exclure celles qui présentent des signes de réversion phénotypique, et
v) la sélection de la souche modifiée parmi les colonies de l'étape iv) comme étant celle qui présente analytiquement une teneur en chlorophylle réduite ou indétectable, telle que définie dans l'une quelconque des revendications 1 à 8,
dans lequel la période de temps de l'étape i) est de préférence suffisante pour pouvoir transférer un échantillon de 10⁷ cellules qui se trouvent en phase de croissance exponentielle vers l'étape ii).
